# EUROPEAN PATENT SPECIFICATION

(11) **EP 3 623 803 B1**
(45) Date of publication and mention of the grant of the patent: **05.11.2025**
(21) Application number: 19192477.8
(22) Date of filing: 20.08.2019
(51) Int. Cl.: G01N 27/04, G01N 33/28, F16H 57/01, F16H 57/04, G01M 13/021, B25J 13/08, B25J 19/00, F01M 11/10, F16H 61/12

(54) **INDUSTRIAL DEVICE INCLUDING A SENSOR FOR SENSING A CONDUCTIVE SUBSTANCE IN A LUBRICANT**
INDUSTRIELLE VORRICHTUNG UMFASSEND EINEN SENSOR ZUR ERFASSUNG EINER LEITFÄHIGEN SUBSTANZ IN EINEM SCHMIERMITTEL
DISPOSITIF INDUSTRIEL COMPRENANT UN CAPTEUR POUR LA DETECTION D'UNE SUBSTANCE CONDUCTRICE DANS UN LUBRIFIANT

(30) Priority: 20.08.2018 JP 2018154140; 30.07.2019 JP 2019139992
(43) Date of publication of application: 18.03.2020
(73) Proprietor: Nabtesco Corporation, Tokyo 102-0093 (JP)
(72) Inventor: NAKAMURA, Koji, Chiyoda-ku, Tokyo 102-0093 (JP); SAKURAI, Kazuhiko, Chiyoda-ku, Tokyo 102-0093 (JP); HARADA, Masaki, Chiyoda-ku, Tokyo 102-0093 (JP); YOTO, Kaoru, Chiyoda-ku, Tokyo 102-0093 (JP); AZUMA, Takahito, Chiyoda-ku, 102-0093 (JP)
(74) Representative: Grünecker Patent- und Rechtsanwälte PartG mbB

(56) References cited:
- EP-A1- 2 808 668
- JP-A- 2010 281 676
- US-A- 4 030 028
- US-A1- 2012 203 509
- US-A1- 2014 347 032
- US-A1- 2017 335 944
- US-A1- 2019 154 608

## Description

### TECHNICAL FIELD

The present invention relates to an industrial device according to independent claim 1.

### BACKGROUND

Such an industrial device is known from US 2017/335944 A1, which is considered to represent the closest prior art. More specifically, this document discloses a gear device in which a sensor is mounted, said sensor including a detection portion configured to detect a foreign substance of a predetermined size or larger floating in a lubricant in a detection space having a boundary at least partially formed by at least one of an outer cylinder and a carrier of the gear device.

A maintenance information output system comprising an iron amount detecting device is known from US 2012/203509 A1. The iron amount detecting device is attached to a speed reducer, and detects an amount of iron in a lubricant in the speed reducer. A proximity sensor is used as the iron quantity detecting device. This proximity sensor transmits the data of the detected iron quantity to a portable wireless receiver via a wireless communicating portion.

EP 2 808 668 A1 describes a machine configured to detect deterioration of lubricant. This machine is equipped with a lubricant deterioration sensor, which includes a light emitting element which is configured to emit light, a color light reception element which is configured to detect color of received light, and a gap forming member having gap forming surfaces at which an oil gap into which the lubricant enters is formed.

A method of and apparatus for detecting the presence of conductive particles in an oil flow system is proposed on US 4 030 028 A. The presence and quantity of conductive materials in the oil flow system are sensed by allowing such materials to settle out of the oil at a point in the system where such oil is relatively static onto a generally planar insulating surface having an array of elongated close spaced substantially parallel electrically conductive surface portions thereon.

An industrial device such as a speed reducer is housed in a housing filled with a lubricating oil so as to prevent damage to mechanical parts such as gears. When the mechanical parts are worn during operation of the industrial device, abrasion powder is mixed into the lubricating oil. An example of the abrasion powder is a conductive substance such as iron powder.

When wear of mechanical parts advances into the wear-out failure period in the failure rate curve (the bathtub curve), a larger amount of abrasion powder is mixed into the lubricating oil. For this reason, a sensor for sensing the amount of the abrasion powder in the lubricating oil can be used for accurate preventive maintenance of the mechanical parts.

As one example of such a sensor, JP 2005 331324 A discloses a sensor that senses the amount of metal powder in an oil. The sensor of Patent Literature 1 includes: a sensor head having a permanent magnet; a cup-shaped electrode provided on a distal end surface of the sensor head; and a plurality of rod-shaped conductive members arranged on an outer peripheral surface of the sensor head. JP 2005 331324 A discloses that an output of the sensor is varied when a short circuit occurs between rod-shaped conductive members due to the abrasion powder accumulated (in a sensing region) between opposed end surfaces of the conductive members and the cup-shaped electrode subjected to a magnetic field by a permanent magnet. This variation of the sensor output indicates a degree of contamination of the oil.

### SUMMARY

Since industrial devices have various sizes from small to large and sizes of mechanical parts in the devices also largely vary, the amount of abrasion powder (conductive substance) produced during operation also varies depending on the devices. In particular, a small industrial device produces a small amount of conductive substance, and therefore, it may be difficult that the conductive substance accumulates on the sensor to an amount necessary for proper failure prediction. Therefore, it is demanded to collect the conductive substance onto the sensor to an amount necessary for failure prediction of the mechanical parts.

One object of the present invention is to provide an industrial device in which abrasion powder (conductive substance) can accumulate on a sensor to an amount necessary for proper failure prediction.

This object is achieved, according to the present invention, by providing an industrial device having the features of independent claim 1. Further advantageous embodiments of the present invention are each the subject of the dependent claims.

An industrial device according to the present invention comprises: a lubricant reservoir portion including a region in which a lubricant circulates; a center shaft member; a center gear configured to rotate about a center axis of the center shaft member; and a sensor including a magnet and a pair of electrodes disposed in the region in which the lubricant circulates, the magnet causing conductive substances contained in the lubricant to be accumulated between the pair of electrodes, and the sensor being configured to sense variation of electric resistance between the pair of electrodes, wherein: the center shaft member has a hollow tubular shape; the center gear is disposed in a vicinity of the sensor; the sensor faces the center gear in a radial direction; a bracket is provided in the lubricant reservoir portion; and the sensor is fixed to the bracket.

The industrial device according to an embodiment of the present invention may further comprise a speed reducer.

The industrial device according to an embodiment of the present invention may further comprise a flange which is a housing member for housing the speed reducer, and a drive source which is attached to the flange.

In the industrial device according to an embodiment of the present invention, the drive source may be a motor.

### ADVANTAGES

One object of the present invention is to provide an industrial device in which abrasion powder (conductive substance) can accumulate on a sensor to an amount necessary for proper failure prediction.

### BRIEF DESCRIPTION OF THE DRAWINGS

Fig. 1 is a sectional view of an industrial device according to a first example not encompassed by the present invention.
Fig. 2 contains a top view and a sectional view of a sensor included in the industrial device of Fig. 1.
Fig. 3 shows a configuration of a sensor according to an embodiment encompassed by the present invention.
Fig. 4 shows a configuration of a sensor according to a second example not encompassed by the present invention.
Fig. 5 shows a configuration of a sensor according to a third example not encompassed by the present invention.
Fig. 6 shows a configuration of a sensor according to a fourth example not encompassed by the present invention.
Fig. 7 shows a configuration of a sensor according to a fifth example not encompassed by the present invention.

### DESCRIPTION OF THE EMBODIMENTS

Embodiments of the present invention will now be described with reference to the appended drawings. Among various devices, a speed reducer will be focused on for convenience of the following description as an example of an industrial device according to embodiments of the invention.

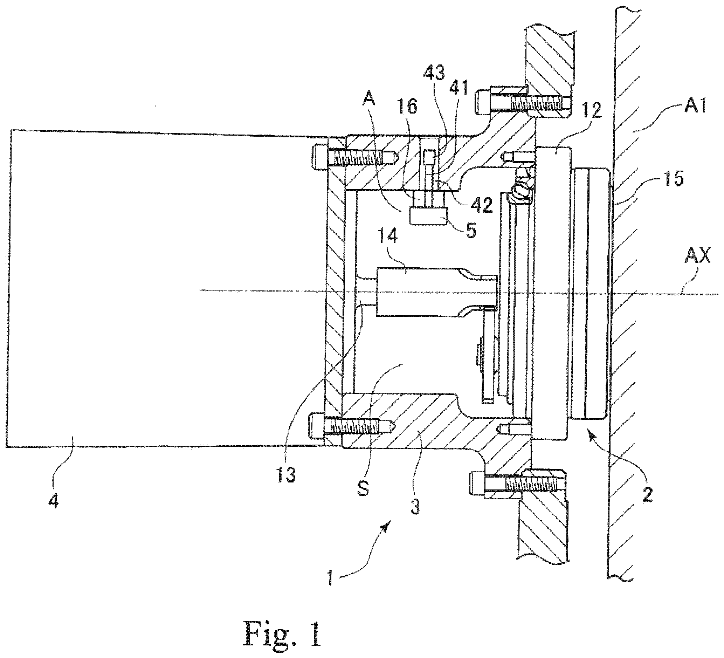

Fig. 1 is a sectional view showing an example of an industrial device 1 according to a first example not encompassed by the present invention. As shown in Fig. 1, the industrial device 1 is a movable part of, e.g., a robot arm. The industrial device 1 includes a speed reducer 2, a flange 3 provided on an input side, a drive source (e.g., a servo motor) 4, and an output-side device A1.

The speed reducer 2 includes a casing 12 mounted to the flange 3, an input shaft 14 connected to an output shaft 13 of the drive source 4, and an output shaft 15 connected to the output-side device A1. The input shaft 14 and the output shaft 15 are supported so as to be rotatable about an axis AX relative to the casing 12. Output of the drive source 4 is input to the speed reducer 2 via the input shaft 14, reduced by the speed reducer 2, and then transmitted to an output-side device A1 via the output shaft 15. Thus, the output-side device A1 and the flange 3 are rotatable relative to each other.

The flange 3 is a tubular member having a hollow portion and houses at least a part of the speed reducer 2. The drive source 4 is mounted to the flange 3. An opening portion in one end of the flange 3 in the direction along the axis AX is closed by the speed reducer 2, and an opening in the other end is closed by the drive source 4. Thus, the flange 3 contains a tightly closed hollow portion (that is, a lubricant reservoir portion) S. The lubricant reservoir portion S contains a lubricant such as a lubricating oil, and the flange 3 also serves as an oil bath.

The casing 12 of the speed reducer 2 houses, for example, a gear mechanism. A space in the casing 12 communicates with the lubricant reservoir portion S in the flange 3. During operation of the speed reducer 2, rotation of the gear mechanism in the casing 12 causes circulation (or convection) of the lubricant between the space in the casing 12 and the space in the flange 3. As the lubricant is circulated, the conductive substance such as abrasion powder produced in the speed reducer 2 is circulated or discharged into the lubricant reservoir portion S in the flange 3. The lubricant reservoir portion S may include a space in the speed reducer 2, in addition to the hollow portion of the flange 3.

In the lubricant reservoir portion S, a sensor 5 for sensing the conductive substance contained in the lubricant is installed. A magnet of the sensor 5 causes the conductive substance contained in the lubricant to be accumulated between a pair of electrodes, and the sensor 5 detects the conductive substance in the lubricant by sensing a change in the electric resistance between the pair of electrodes. For example, the sensor 5 is installed in a region A shown in Fig. 1, where the lubricant circulates.

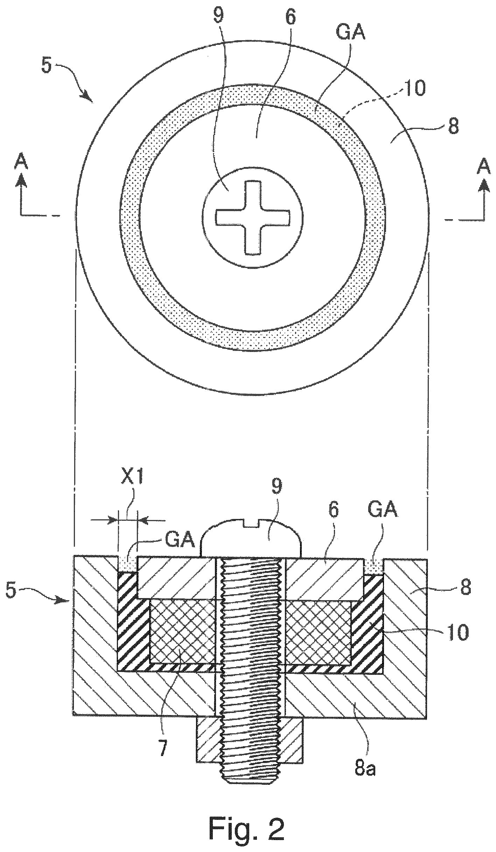

Next, with reference to Fig. 2, a description is given of an example of the sensor 5. The sensor 5, which may have various shapes, structures, and sizes, is not limited to the aspect shown in the drawing. Fig. 2 shows a configuration of the sensor 5 installed in the industrial device according to an embodiment of the present invention. Fig. 2 contains a top view of the sensor 5 and a sectional view along the line A-A of the top view.

As shown in Fig. 2, the sensor 5 includes a center electrode (first electrode) 6, a magnet 7, a box-shaped electrode (second electrode) 8, a screw 9, and a resin material 10. As shown in the drawing, the center electrode (first electrode) 6 and the magnet 7 are fixed to the box-shaped electrode (second electrode) 8 by the screw 9. A signal line 41 shown in Fig. 1 is connected to the box-shaped electrode 8, and a signal line 42 is connected to the center electrode 6.

The box-shaped electrode 8 is a magnetic member formed of a magnetic material having electric conductivity such as iron, ferrite, or silicon steel. The box-shaped electrode 8 has a substantially cylindrical shape and its opening is closed on one end in the axial direction (the bottom in the sectional view of Fig. 2) by a bottom portion 8a. Thus, the box-shaped electrode 8 has a cylindrical box-like shape with an opening at the top. Alternatively, the shape of the box-shaped electrode 8 may be a rectangular parallelepiped with an opened top or a polygonal tube with a closed bottom.

In the box-shaped electrode 8, there is disposed a resin material 10, which is a non-magnetic material (an insulator). In this manner, the center electrode (first electrode) 6 and the magnet 7 are formed such that at least a part of them are buried in the central region of the resin material. The box-shaped electrode 8 is arranged so as to surround the magnet 7 and the resin material 10. The shapes of the magnet 7 and the center electrode 6 are not limited to cylindrical shapes but may be a rectangular parallelepiped, a polygonal column, or the like.

As shown in the sectional view of Fig. 2, the outer shape of the center electrode 6 is smaller than the inner periphery of the box-shaped electrode 8. Accordingly, a gap GA is formed between the center electrode 6 and the box-shaped electrode 8 over the entire periphery of the center electrode 6 (so as to surround the center electrode). In other words, the center electrode 6 and the box-shaped electrode 8 are arranged so as to face each other with the gap GA on the resin material 10 interposed therebetween. The gap GA is formed on the resin material 10.

Output lines (signal lines 41, 42 shown in Fig. 1) are connected to the center electrode 6 and the box-shaped electrode 8, respectively. As shown in the drawing, the magnet 7 is either attached or not attached to the bottom of the center electrode 6. In addition, when the magnet 7 is attached, the magnet 7, which is formed of magnet or an electromagnet, may be coated with a nonmagnetic material such as copper, and the signal line 41 or the signal line 42 may be connected to the coating layer . When the magnet 7 is formed of an electromagnet, the center electrode 6 may also serve as an electrode for the electromagnet.

The output lines are connected, at the output ends thereof, to a sensor driving circuit (not shown). The sensor driving circuit monitors a resistance value of the sensor 5 to predict a failure of the mechanical parts based on variation of the resistance value due to the accumulation of the conductive substance between the electrodes. When a certain amount of conductive substance accumulates in the gap GA, the electric resistance between the center electrode 6 to which a voltage is applied and the box-like electrode 8 decreases, and output levels of the output lines change. The sensor driving circuit detects the decrease in the electric resistance to predict a failure of the mechanical parts. The decrease in the electric resistance may be indicated with an On signal (with electricity passing) and an Off signal (with no electricity passing), so that it is possible to perform sensing between these On and Off states (hereinafter referred to as "digital sensing").

The sensor driving circuit is connected to a superior control device such as a manipulator in a wired or wireless manner. A circuit board 43 in Fig. 1 may transmit outputs of the output lines (an output of the sensor 5) to a superior control device either constantly or intermittently (at regular time intervals) for saving electricity.

When sensing the variation of the output levels of the output lines received from the circuit board 43, the superior control device may give an alert for demanding maintenance of, for example, the speed reducer 2 with a predetermined notification means (a display or a voice output device).

The magnet 7 is magnetized to form a magnetic flux path φA (not shown) in a predetermined direction. In particular, a strong magnetic flux flows in the gap GA around the center electrode 6. By a magnetic force of the magnet 7, the conductive substance from mechanical parts (for example, the conductive substance from mechanical parts mixed in the lubricating oil) is attracted into the gap GA.

Here, as wear of mechanical parts advances into the wear-out failure period in the failure rate curve (the bathtub curve), the amount of produced conductive substance increases, but this amount greatly differs depending on the size of the industrial device. In general, a larger device contains a larger number of mechanical parts or mechanical parts of a larger size, and therefore, more conductive substance is produced. By contrast, a small device produces a small amount of conductive substance, and therefore, it may be difficult that the conductive substance accumulates on the sensor to an amount necessary for proper failure prediction. Therefore, it is preferable to collect the conductive substance onto the sensor 5 to an amount necessary for failure prediction of the mechanical parts.

As described above, the industrial device 1 includes the sensor 5 installed in the region A where the lubricant is circulated. Thus, the conductive substance contained in the lubricant tends to pass by the sensor 5, so as to be attracted toward the sensor 5. Accordingly, the abrasion powder (conductive substance) can accumulate on the sensor 5 to an amount necessary for proper failure prediction.

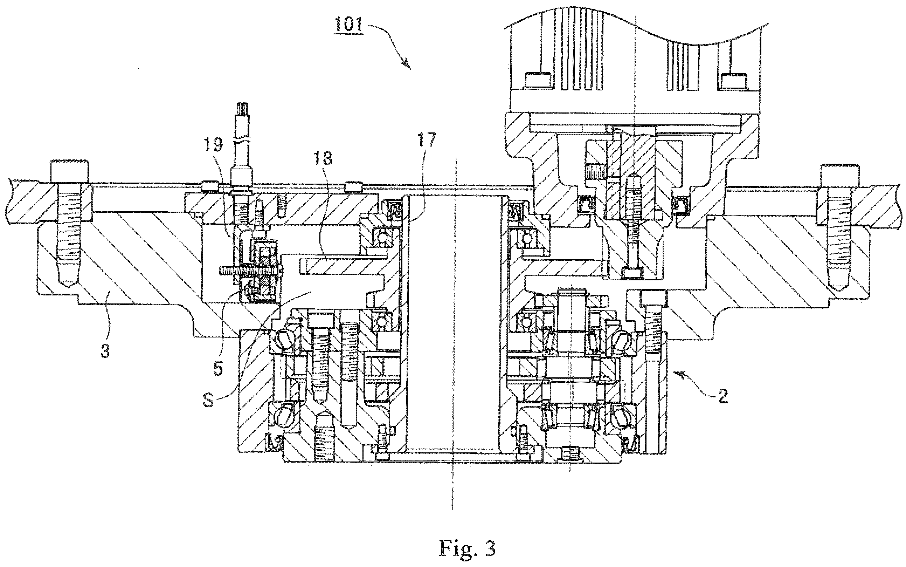

Next, an industrial device 101 according to an embodiment encompassed by the present invention will be described with reference to Fig. 3. The industrial device 101 includes a flange 3, and at least a part of a speed reducer 2 is housed in the flange 3. The flange 3 is a housing member for housing the speed reducer 2, and a drive source (e.g., a motor) 4 is attached to the flange 3. The flange 3 is a substantially tubular member having a hollow portion (a lubricant reservoir portion S). The lubricant reservoir portion S contains a lubricant (e.g., a lubricating oil).

As with the industrial device 1 shown in Fig. 1, the industrial device 101 includes: the lubricant reservoir portion S for containing the lubricant; and the sensor 5 having a pair of electrodes (that is, a first electrode 6 and a second electrode 8) and configured to apply a voltage between the pair of electrodes and allow accumulation of the conductive substance between the electrodes, thereby to sense variation of electric resistance between the pair of electrodes.

The industrial device 101 includes a hollow tubular member 17 and a center gear 18 that rotates about the center axis of the tubular member 17. The sensor 5 is disposed in the vicinity of the center gear 18.

Since the sensor 5 is disposed in the vicinity of the center gear 18, the rotation of the center gear 18 causes the lubricant to circulate, such that a larger amount of conductive substance can be collected. Accordingly, the abrasion powder (conductive substance) can accumulate on the sensor 5 to an amount necessary for proper failure prediction.

In the industrial device 101, the sensor 5 is installed in a region facing the center gear 18. Thus, the rotation of the center gear 18 can cause the lubricant to circulate toward the sensor, such that a larger amount of conductive substance can be collected. Accordingly, the abrasion powder (conductive substance) can accumulate on the sensor 5 to an amount necessary for proper failure prediction.

The industrial device 101 further includes a bracket 19 in the lubricant reservoir portion S, and the sensor 5 is fixed to the bracket 19. Thus, the sensor is positioned to face the center gear 18, such that a larger amount of conductive substance can be collected. Accordingly, the abrasion powder (conductive substance) can accumulate on the sensor 5 to an amount necessary for proper failure prediction. The bracket 19 may be mounted with a screw to an appropriate location in the industrial device 1, but the mounting method can be changed if necessary.

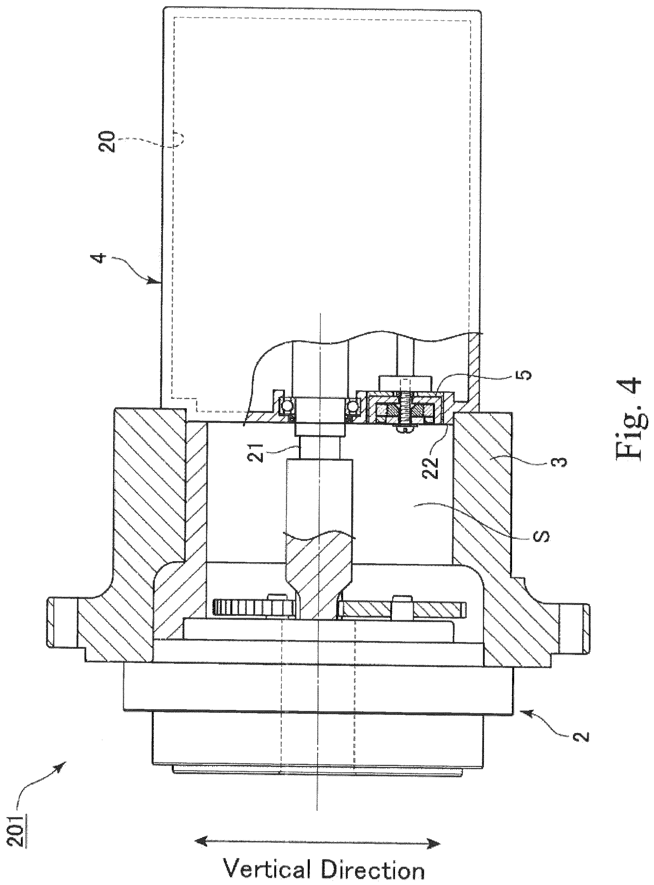

Next, an industrial device 201 according to a second example not encompassed by the present invention will be described with reference to Fig. 4. The industrial device 201 includes a flange 3, and at least a part of a speed reducer 2 is housed in the flange 3. The flange 3 is a housing member for housing the speed reducer 2, and a drive source (e.g., a motor) 4 is attached to the flange 3. The flange 3 is a substantially tubular member having a hollow portion (a lubricant reservoir portion S). The lubricant reservoir portion S contains a lubricant (e.g., a lubricating oil).

The industrial device 201 includes: the lubricant reservoir portion S for containing the lubricant; and the sensor 5 having a pair of electrodes (that is, a first electrode 6 and a second electrode 8) and configured to apply a voltage between the pair of electrodes and allow accumulation of the conductive substance between the pair of electrodes, thereby to sense variation of electric resistance between the pair of electrodes.

The industrial device 201 includes a drive source 4 that is partially housed in a casing 20. For example, the drive source 4 is a motor. One end portion of the lubricant reservoir portion S is defined by an end surface 22 of the casing 20. A drive shaft 21 of the drive source 4 extends through the end surface 22 of the casing 20, and the sensor 5 is fixed to the end surface 22 of the casing 20. Thus, the rotation of the drive shaft 21 of the drive source 4 can cause the lubricant to circulate toward the sensor 5, such that a larger amount of conductive substance can be collected. Accordingly, the abrasion powder (conductive substance) can accumulate on the sensor 5 to an amount necessary for proper failure prediction. In addition, as compared to the case where the sensor 5 is provided on the flange 3, the flange 3 can be shaped more simply, and the connection line of the drive source 4 and the connection line of the sensor 5 can be disposed together more easily.

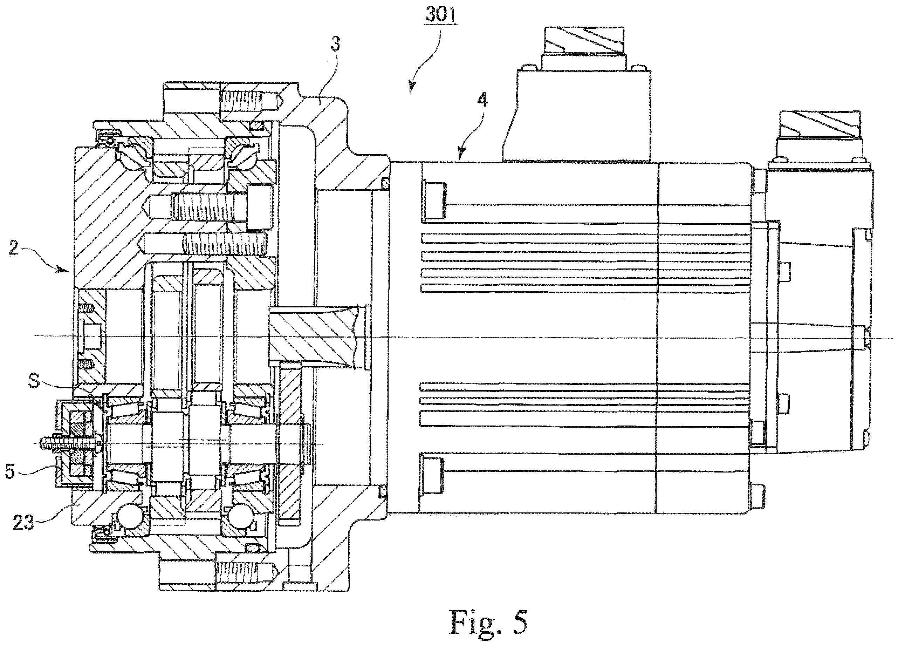

Next, an industrial device 301 according to a third example not encompassed by the present invention will be described with reference to Fig. 5. The industrial device 301 includes a flange 3, and at least a part of a speed reducer 2 is housed in the flange 3. The flange 3 is a housing member for housing the speed reducer 2 including a carrier 23, and a drive source (e.g., a motor) 4 is attached to the flange 3. The flange 3 is a substantially tubular member having a hollow portion (the lubricant reservoir portion S). The lubricant reservoir portion S contains a lubricant (e.g., a lubricating oil).

The industrial device 301 includes: the lubricant reservoir portion S for containing the lubricant; and the sensor 5 having a pair of electrodes (that is, a first electrode 6 and a second electrode 8) and configured to apply a voltage between the pair of electrodes and allow accumulation of the conductive substance between the pair of electrodes, thereby to sense variation of electric resistance between the pair of electrodes.

The industrial device 1 according to this example includes the carrier 23 that is rotatable, and the sensor 5 is fixed to the carrier 23. The sensor 5 rotates with the carrier 23, and thus sensor 5 contacts with much lubricant while moving. Accordingly, the abrasion powder (conductive substance) can accumulate on the sensor 5 to an amount necessary for proper failure prediction.

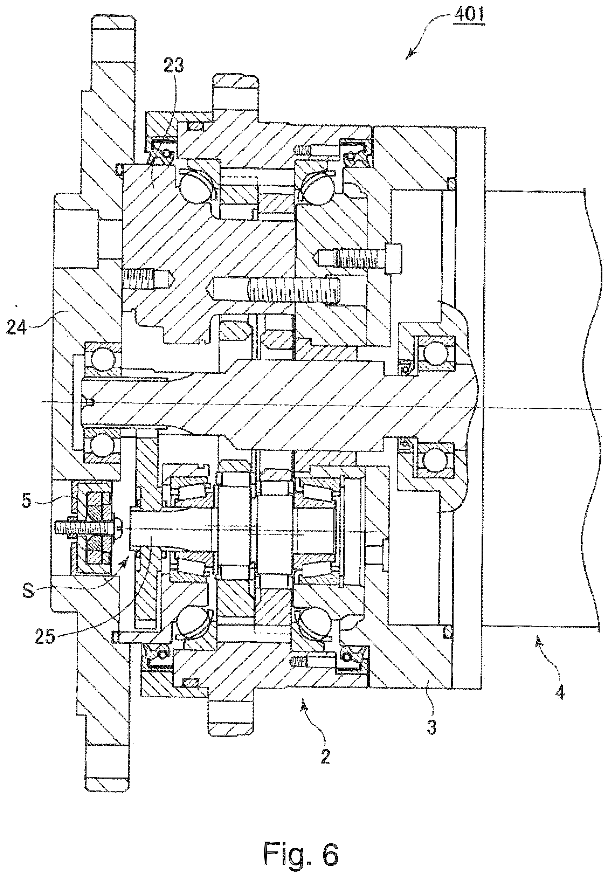

Next, an industrial device 401 according to a fourth example not encompassed by the present invention will be described with reference to Fig. 6. The industrial device 401 includes a flange 3, and at least a part of a speed reducer 2 including a carrier 23 is housed in the flange 3. On the output side of the speed reducer 2, the carrier 23 is connected to a first member (e.g., a robot arm) 24. The flange 3 is a housing member for housing the speed reducer 2, and a drive source (e.g., a motor) 4 is attached to the flange 3. The flange 3 is a substantially tubular member having a hollow portion (a lubricant reservoir portion S). The lubricant reservoir portion S contains a lubricant (e.g., a lubricating oil).

The industrial device 401 includes: the lubricant reservoir portion S for containing the lubricant; and the sensor 5 having a pair of electrodes (that is, a first electrode 6 and a second electrode 8) and configured to apply a voltage between the pair of electrodes and allow accumulation of the conductive substance between the pair of electrodes, thereby to sense variation of electric resistance between the pair of electrodes.

The industrial device 401 includes the carrier 23 connected to the first member 24. The first member 24 is, for example, a robot arm. The speed reducer 2 and the members attached thereto can rotate relative to the flange 3, and the sensor 5 is fixed to the first member 24. The sensor 5 rotates with the first member 24, and thus sensor 5 contacts with much lubricant while moving. Accordingly, the abrasion powder (conductive substance) can accumulate on the sensor 5 to an amount necessary for proper failure prediction.

The industrial device 401 includes a crank member (crank mechanism) 25, and the sensor is fixed at such a position as to face the crank member 25. The sensor 5 is fixed at such a position as to face the crank member 25 in the axial direction of the crank member 25. Thus, the sensor 5 contacts with much lubricant while moving in the vicinity of the crank member 25, such that a larger amount of conductive substance can be collected. Accordingly, the abrasion powder (conductive substance) can accumulate on the sensor 5 to an amount necessary for proper failure prediction.

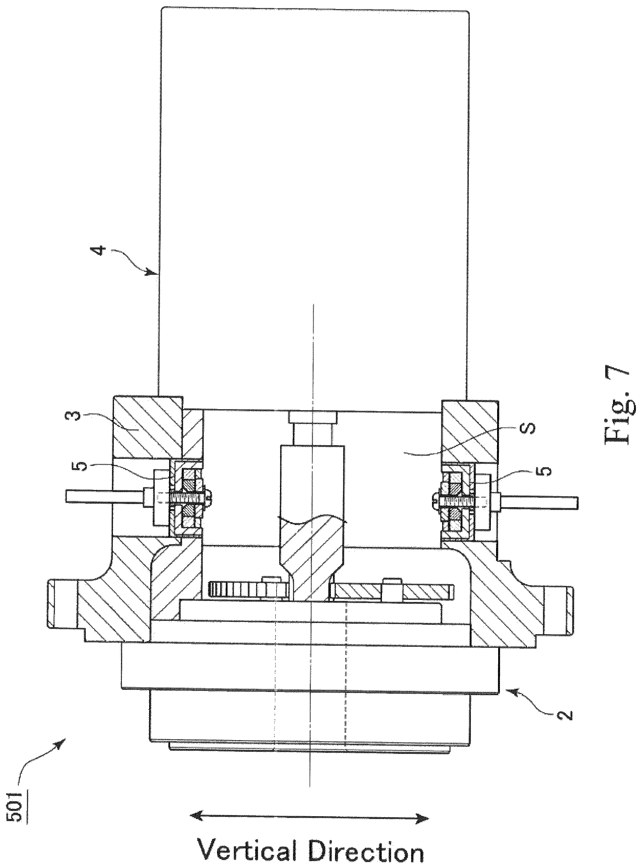

Next, an industrial device 501 according to a fifth example not encompassed by the present invention will be described with reference to Fig. 7. The industrial device 501 includes a flange 3, and at least a part of a speed reducer 2 is housed in the flange 3. The flange 3 is a housing member for housing the speed reducer 2, and a drive source (e.g., a motor) 4 is attached to the flange 3. The flange 3 is a substantially tubular member having a hollow portion (a lubricant reservoir portion S). The lubricant reservoir portion S contains a lubricant (e.g., a lubricating oil).

The industrial device 501 includes: the lubricant reservoir portion S for containing the lubricant; and the sensor 5 having a pair of electrodes (that is, a first electrode 6 and a second electrode 8) and configured to apply a voltage between the pair of electrodes and allow accumulation of the conductive substance between the pair of electrodes, thereby to sense variation of electric resistance between the pair of electrodes.

In the industrial device 501, the sensor 5 is positioned vertically below the lubricant reservoir portion S. The industrial device 501 is disposed such that, for example, a drive shaft of the drive source 4 is orthogonal to the vertical direction. The sensor 5, which is disposed below the oil bath, readily collects the conductive substance precipitated due to insufficient stirring of the lubricant, facilitating the failure prediction of the industrial device 1. Since the precipitated conductive substance can be readily attracted, the abrasion powder (conductive substance) can accumulate on the sensor 5 to an amount necessary for proper failure prediction.

The industrial device 501 includes another sensor 5 having a pair of electrodes and configured to allow accumulation of the conductive substance between the pair of electrodes, thereby to sense variation of electric resistance between the pair of electrodes. The other sensor 5 is positioned vertically above the lubricant reservoir portion S. When stirring of the lubricant is insufficient, conductive substance produced upon a failure of the speed reducer precipitates on the sensor 5 disposed above the oil bath. In this arrangement, the sensor 5 disposed above the oil bath, which receives less conductive substance accumulated thereon, reacts a certain period of time after the failure of the speed reducer (more conductive substance is distributed after the failure). Therefore, it is possible that the sensor 5 disposed vertically below the oil bath that more readily collects conductive substance precipitated thereon is used for failure prediction, and the sensor 5 disposed vertically above the oil bath detects a large amount of conductive substance distributed upon a failure to detect the failure, thus achieving less erroneous sensing operation. With further sensors 5 added, a sensor group can be constituted that is capable of more reliable and accurate sensing.

### LIST OF REFERENCE NUMBERS

- 1: industrial device
- 2: speed reducer
- 3: flange portion
- 4: drive source
- 5: sensor
- 6: center electrode
- 7: magnet
- 8: box-shaped electrode
- 9: screw
- 10: resin material
- 12: casing
- 13: output shaft
- 14: input shaft
- 15: output shaft
- 17: tubular member
- 18: center gear
- 19: bracket
- 20: casing
- 21: drive shaft
- 22: end surface
- 23: carrier
- 24: first member
- 25: crank member
- 41: signal line
- 42: signal line
- 43: circuit board

## Claims

1. An industrial device (1), comprising:
a lubricant reservoir portion (S) including a region in which a lubricant circulates;
a center shaft member (17);
a center gear (18) configured to rotate about a center axis of the center shaft member (17); and
a sensor (5) including a magnet (7) and a pair of electrodes (6, 8) disposed in the region in which the lubricant circulates, the magnet (7) causing conductive substances contained in the lubricant to be accumulated between the pair of electrodes (6, 8), and the sensor (5) being configured to sense variation of electric resistance between the pair of electrodes (6, 8), wherein:
the center shaft member (17) has a hollow tubular shape;
the center gear (18) is disposed in a vicinity of the sensor (5); and
the sensor (5) faces the center gear (18) in a radial direction,
**characterized in that**
a bracket (19) is provided in the lubricant reservoir portion (S),
wherein the sensor (5) is fixed to the bracket (19).

2. The industrial device (1) of claim 1, further comprising a speed reducer (2).

3. The industrial device (1) of claim 2, further comprising:
a flange (3) which is a housing member for housing the speed reducer (2); and
a drive source (4) which is attached to the flange (3).

4. The industrial device (1) of claim 3, wherein the drive source (4) is a motor.

## Patentansprüche

1. Eine Industrievorrichtung (1), aufweisend:
einen Schmiermittelreservoirabschnitt (S), der einen Bereich aufweist, in dem ein Schmiermittel zirkuliert;
ein Mittelwellenelement (17);
ein Mittelzahnrad (18), das so konfiguriert ist, dass es sich um eine Mittelachse des Mittelwellenelements (17) dreht; und
einen Sensor (5), der einen Magneten (7) und ein Paar von Elektroden (6, 8) aufweist, die in dem Bereich angeordnet sind, in dem das Schmiermittel zirkuliert, wobei der Magnet (7) bewirkt, dass sich in dem Schmiermittel enthaltene leitfähige Substanzen zwischen dem Paar von Elektroden (6, 8) ansammeln, und der Sensor (5) so konfiguriert ist, dass er eine Änderung des elektrischen Widerstands zwischen dem Paar von Elektroden (6, 8) erfasst, wobei:
das Mittelwellenelement (17) eine rohrförmige Hohlform aufweist;
das mittlere Zahnrad (18) in der Nähe des Sensors (5) angeordnet ist; und
der Sensor (5) dem mittleren Zahnrad (18) in einer radialen Richtung gegenüberliegt,
**dadurch gekennzeichnet, dass**
eine Halterung (19) in dem Schmiermittelreservoirabschnitt (S) vorgesehen ist,
wobei der Sensor (5) an der Halterung (19) befestigt ist.

2. Die Industrievorrichtung (1) nach Anspruch 1, die weiterhin einen Drehzahlreduzierer (2) aufweist.

3. Die Industrievorrichtung (1) nach Anspruch 2, die ferner aufweist:
einen Flansch (3), der ein Gehäuseelement zur Aufnahme des Drehzahlreduzierers (2) ist; und eine Antriebsquelle (4), die an dem Flansch (3) befestigt ist.

4. Die Industrievorrichtung (1) nach Anspruch 3, wobei die Antriebsquelle (4) ein Motor ist.

## Revendications

1. Dispositif industriel (1) comportant :
une partie réservoir de lubrifiant (S) incluant une région dans laquelle circule un lubrifiant ;
un élément d'arbre central (17) ;
une roue dentée centrale (18) configurée pour tourner autour d'un axe central de l'élément d'arbre central (17) ; et
un capteur (5) incluant un aimant (7) et une paire d'électrodes (6, 8) disposées dans la région dans laquelle circule le lubrifiant, l'aimant (7) amenant des substances conductrices contenues dans le lubrifiant à s'accumuler entre la paire d'électrodes (6, 8), et le capteur (5) étant configuré pour détecter une variation de résistance électrique entre la paire d'électrodes (6, 8), où :
l'élément d'arbre central (17) a une forme tubulaire creuse ;
la roue dentée centrale (18) est disposée à proximité du capteur (5) ; et
le capteur (5) fait face à la roue dentée centrale (18) dans une direction radiale,
**caractérisé en ce que**
un support (19) est fourni dans la partie réservoir de lubrifiant (S),
où le capteur (5) est fixé au support (19).

2. Dispositif industriel (1) de la revendication 1, comportant en outre un réducteur de vitesse (2).

3. Dispositif industriel (1) de la revendication 2, comportant en outre :
une bride (3) qui est un élément de logement pour loger le réducteur de vitesse (2) ; et une source d'entraînement (4) qui est attachée à la bride (3).

4. Dispositif industriel (1) de la revendication 3, où la source d'entraînement (4) est un moteur.
